# EUROPEAN PATENT APPLICATION

(11) **EP 3 795 028 A1**
(43) Date of publication of application: **24.03.2021**
(21) Application number: 19804578.3
(22) Date of filing: 15.05.2019
(51) Int. Cl.: A44C 5/02, A44C 5/00, A61B 5/0245

(54) **RUBBER BAND MANUFACTURING METHOD, RUBBER BAND, AND ELECTRONIC APPARATUS TO BE ATTACHED TO HUMAN BODY**

(30) Priority: 15.05.2018 JP 2018093970
(71) Applicant: Sekisui Polymatech Co., Ltd., Saitama-city, Saitama 338-0837 (JP)
(72) Inventor: KAWAGUCHI Asei William, Saitama-city, Saitama 338-0837 (JP)
(74) Representative: Schmid, Wolfgang
(86) International application number: PCT/JP2019/019385
(87) International publication number: WO 2019/221199

(57) **Abstract**

[Abstract]

Provided is an improved rubber band for mounting an electronic device body on a body. In a rubber band 10 and in a method for manufacturing a rubber band 10, a resin plate having a plurality of resin holes 5d is inserted between an outer-surface mold 8a and a back mold 8b to form a first outer sheath 6a on a surface 5A of the resin plate. Subsequently, the resin plate on which the first outer sheath 6a is formed is inserted between the outer-surface mold 8a and an inner-surface mold 8b to form a second outer sheath 6b on a back surface 5B of the resin plate.

## Description

### Technical Field

The present application relates to a rubber band for an electronic device used by being wrapped around a human body such as an arm or wrist for fixing the device, a method for manufacturing the same, and an electronic device including the rubber band.

### Background Art

Some electronic devices include a band that is wrapped around a human body such as an arm or wrist to fix the device. Examples of electronic devices that include a band include a watch, a portable terminal device, a medical electronic device (such as a pulsimeter or a sphygmomanometer), and a health management electronic device (such as a pulsimeter or a sphygmomanometer). Bands used for these electronic devices include metal-made and resin-made bands. A resin-made band is described in, for example, Japanese Unexamined Patent Application Publication No. 2011-72777 (PTL 1).

### Citation List

### Patent Literature

PTL 1: Japanese Unexamined Patent Application Publication No. 2011-72777

### Summary of Invention

### Technical Problem

Among electronic devices including metal-made or resin-made bands, a medical electronic device or health management electronic device needs to obtain accurate measurement results, such as the pulse or blood pressure, from a human body. Here, for example, an electronic device serving as a watch may be loosely wound around the wrist depending on wearer's preferences. A medical electronic device or health management electronic device (including a watch having these functions), on the other hand, is required to bring a sensor unit, which measures biological information, into contact with the body. To bring the sensor unit into contact with the body, the band has to be fastened to the body. On the other hand, for a wearer to feel a comfortable fitting while wearing the electronic device, the band is to be less tightly fastened.

The band has a large number of band length adjustment holes to allow a wearer to adjust the strength by which the band is fastened to the wrist. The wearer can adjust the degree of contact of the sensor unit with the body when the wearer wears the band by selecting which of the holes he/she inserts a buckle tongue. However, metal-made and resin-made bands, which are made of a nonstretchable material, hardly ever achieve adhesion properties and comfort even by reducing the intervals between band length adjustment holes adjacent to each other. A rubber band, on the other hand, can stretch as appropriate and has a soft texture, so that the rubber band can achieve both adhesion properties and comfort. However, the rubber band is weaker than other materials, and thus easily elongated and easily cut. Particularly, when the intervals between the band length adjustment holes adjacent to each other, into which the buckle tongue is inserted, are reduced to allow a wearer to easily adjust the degree of contact to the wrist when the wearer wears the band, the intervals are more likely to be cut. In addition, excessive elasticity prevents stable contact of a sensor to the human body although a small degree of elasticity is required for accurate measurement. In addition, such an electronic device is required to be worn all the time, and the band is thus required to have excellent design.

The disclosure of the present application aims to provide a rubber band improved to mount an electronic device on the body.

### Solution to Problem

To achieve the above object, the present invention provides the following structure. Specifically, the present invention provides a method for manufacturing a rubber band including a band body attached to the electronic device body and worn by a human body. The band body includes a resin portion formed from a resin plate having a plurality of holes, an outer sheath made of a rubber-like elastic material that covers the resin portion, and a plurality of band length adjustment holes that extend through the plurality of holes of the resin portion and the outer sheath. The resin plate having the plurality of holes (or "resin holes") is inserted into a first mold to form a first outer sheath covering a first surface of the resin plate. Then, the resin plate on which the first outer sheath is disposed is inserted into a second mold to form a second outer sheath covering a second surface of the resin plate.

In the present invention, the resin plate having a plurality of holes is inserted into the first mold to form a first outer sheath that covers the first surface of the resin plate, and then the resin plate on which the first outer sheath is disposed is inserted into the second mold to form the second outer sheath that covers the second surface of the resin plate. Thus, a resin portion is easily inserted between the first outer sheath and the second outer sheath, and easily placed at an intended position between the first outer sheath and the second outer sheath. Thus, the present invention can provide a rubber band including the resin portion formed from a rubber-like elastic material that can reinforce the first outer sheath and the second outer sheath.

The outer sheath (first outer sheath and second outer sheath) is formed from a rubber-like elastic material. Thus, the softness of the rubber-like elastic material can enhance well-fitted feeling to the skin, and the elasticity of the rubber-like elastic material can change the degree of contact to the body, so that the rubber band can fix the sensor unit to the body. The rubber band holds the resin portion inside. Thus, the rubber-like elastic material can be prevented from being excessively elongated, and protected against being cut or broken.

According to the present invention, when forming the first outer sheath or the second outer sheath, the band length adjustment holes can be formed by covering an inner peripheral surface of each of the plurality of holes with the rubber-like elastic material. When forming the first outer sheath or the second outer sheath, the band length adjustment holes are formed by covering the inner peripheral surface of each of the plurality of holes with the rubber-like elastic material. The resin portion covered with the rubber-like elastic material is thus disposed around the band length adjustment holes. The rubber band can thus have the portions between the band length adjustment holes reinforced with the resin portion. The rubber band can thus have the band length adjustment holes less easily breakable even though a gap between the band length adjustment holes is narrow. The resin portion is hidden at the band length adjustment holes. Thus, the rubber band can have an excellent appearance. As a specific example of a method for manufacturing a rubber band, the band length adjustment holes may be formed by covering half the inner peripheral surfaces of the plurality of holes with the rubber-like elastic material from either surface of the resin portion when forming each of the first outer sheath and the second outer sheath.

According to the present invention, the resin plate may include a flat plate-shaped body, a cylinder formed by winding an end portion of the body, and an extra length portion extended from the cylinder and layered on the body, and when forming each of the first outer sheath and the second outer sheath, an outer peripheral surface of the cylinder may be partially covered.

The resin plate includes a flat plate-shaped body, a cylinder formed by winding a first end portion of the body, and an extra length portion extended from the cylinder and layered on the body. Thus, the cylinder can reinforce the periphery of the spring-bar receiving hole. Thus, the rubber band can have a less cuttable spring-bar receiving hole. In the resin plate, the cylinder can serve as a positioning portion for molding. The resin plate includes an extra length portion. Thus, stably forming the cylinder enhances the reinforcing property of the spring-bar receiving hole. When forming each of the first outer sheath and the second outer sheath, the outer peripheral surface of the cylinder is partially covered. Thus, a portion of the outer sheath around the cylinder, that is, a portion of the outer sheath forming the spring-bar receiving hole can be accurately molded. Thus, the rubber band having an excellent appearance can be well integrated with the electronic device body when attached to the electronic device body.

According to the present invention, when forming the first outer sheath or the second outer sheath, an inner peripheral surface of the cylinder may be covered with the rubber-like elastic material. Thus, the obtained rubber band may cover the inner periphery of the spring-bar receiving hole with the rubber-like elastic material. Thus, the spring-bar receiving hole and the spring bar come into contact with each other while having appropriate friction resistance therebetween. The rubber band is not excessively unfastened with respect to the electronic device body, and the electronic device body and the rubber band may thus be integrated to form an electronic device.

According to the present invention, the first mold and the second mold receive an uncured rubber-like elastic material and the resin portion to mold them into a particular shape. Unlike injection molding, the present invention has no need to consider an inlet passage and an outlet passage for a molten rubber-like elastic material, and the rubber-like elastic material easily flows throughout inside the first mold and the second mold while being less likely to cause defects such as a liquid pool.

The present invention aiming to attain the above object further provides a rubber band that includes a first band body attached to an electronic device body and worn by a human body. The first band body includes a plate-shaped resin portion having a plurality of holes, and an outer sheath made of a rubber-like elastic material that covers the resin portion. The outer sheath includes an outer surface portion that covers a first surface of the resin portion, and an inner surface portion that covers a second surface of the resin portion and comes into contact with a human body. The outer surface portion and the inner surface portion are formed as separate bodies. The first band body further includes a plurality of band length adjustment holes into which the lock portion is inserted. Each of the band length adjustment holes has an inner-band-length-adjustment-hole cover formed by covering an inner peripheral surface of each band length adjustment hole with the outer sheath.

The rubber band includes the rubber-like elastic material reinforced with the resin portion to be made less cuttable, and is prevented from being excessively elongated to reliably obtain data. The rubber band has the resin portion covered with the first outer sheath and the second outer sheath, and thus provides reliable contact with the body and excellent texture to the skin. The rubber band having the resin portion covered with the outer sheath has a fine outer appearance design.

The rubber band further includes a second band body different from the first band body. The second band body includes a lock portion that is locked with the first band body.

The rubber band includes a second band body that includes a lock portion that is locked with the first band body. Thus, the first band body and the second band body may be connected with the lock portion. Here, the second band body may be provided as a body separate from the first band body. Instead, the first band body and the second band body may be connected across the electronic device body to form an integrated band body.

The resin portion may include a flat plate-shaped body, a cylinder formed by winding an end portion of the body, and an extra length portion extended from the cylinder and layered on the body. Thus, a rubber band having the periphery of the spring-bar receiving hole reinforced with the cylinder and the spring-bar receiving hole made less cuttable may be obtained. The rubber band having the extra length portion enables stable formation of the cylinder, and has a less breakable spring-bar receiving hole.

The outer sheath includes an inner-cylinder cover that covers an inner peripheral surface of the cylinder. The rubber band includes the inner periphery of the spring-bar receiving hole covered with the rubber-like elastic material. The inner-cylinder cover allows the spring-bar receiving hole and the spring bar to come into contact with each other while having appropriate friction resistance therebetween, and prevents the rubber band from being excessively unfastened with respect to the electronic device body. Thus, the electronic device body and the rubber band may be integrally formed into an electronic device.

The present invention may be embodied as a wearable electronic device that includes an electronic device body, and the rubber band according to any one of the above examples. The electronic device is less likely to tire a wearer from long term wearing, is capable of being in close contact with the human body, is capable of reliably obtaining data, has durability, and has excellent outer appearance. Advantageous Effects of Invention

The present invention can embody a rubber band that can appropriately bring an electronic device into contact with the body. The present invention can also embody an electronic device including such a rubber band.

### Brief Description of Drawings

[Fig. 1] Fig. 1 is a schematic plan view of an electronic device formed from a rubber band according to a first embodiment attached to an electronic device body.
[Fig. 2] Fig. 2 is a plan view of the rubber band illustrated in Fig. 1.
[Fig. 3] Fig. 3 is a front view of the rubber band illustrated in Fig. 1.
[Fig. 4] Fig. 4 is an enlarged cross-sectional view of a point-side band body of the rubber band illustrated in Fig. 1, taken along line IV-IV in Fig. 2.
[Fig. 5] Fig. 5 illustrates a resin portion illustrated in Fig. 4, where Fig. 5(A) is a cross-sectional view corresponding to Fig. 4, and Fig. 5(B) is a plan view.
[Fig. 6] Fig. 6 is an enlarged cross-sectional view of a buckle-side band body of the rubber band illustrated in Fig. 1, taken along line VI-VI in Fig. 2.
[Fig. 7] Fig. 7 illustrates a resin portion illustrated in Fig. 6, where Fig. 7(A) is a cross-sectional view corresponding to Fig. 6, and Fig. 7(B) is a plan view.
[Fig. 8] Fig. 8 is a schematic cross-sectional view illustrating a first step of a method for manufacturing the rubber band illustrated in Fig. 1.
[Fig. 9] Fig. 9 is a schematic cross-sectional view illustrating a second step of a method for manufacturing the rubber band illustrated in Fig. 1.
[Fig. 10] Fig. 10 is a partially enlarged cross-sectional view around resin holes in the first step of the method for manufacturing the rubber band illustrated in Fig. 1.
[Fig. 11] Fig. 11 is an enlarged cross-sectional view around the resin holes in the second step of the method for manufacturing the rubber band illustrated in Fig. 1.
[Fig. 12] Fig. 12 is a partially enlarged cross-sectional view around the resin holes in the rubber band illustrated in Fig. 1.
[Fig. 13] Fig. 13 is a schematic plan view of a rubber band according to a second embodiment.
[Fig. 14] Fig. 14 is a schematic plan view of a rubber band according to a third embodiment.

### Description of Embodiments

The present invention will be described further in detail using embodiments. In each embodiment, the same material, composition, method, operation, effects, and other features will not be described redundantly. The terms "first" and "second" in the description and the scope of claims are used to distinguish different components of the present invention, not to indicate, for example, a specific order or superiority.

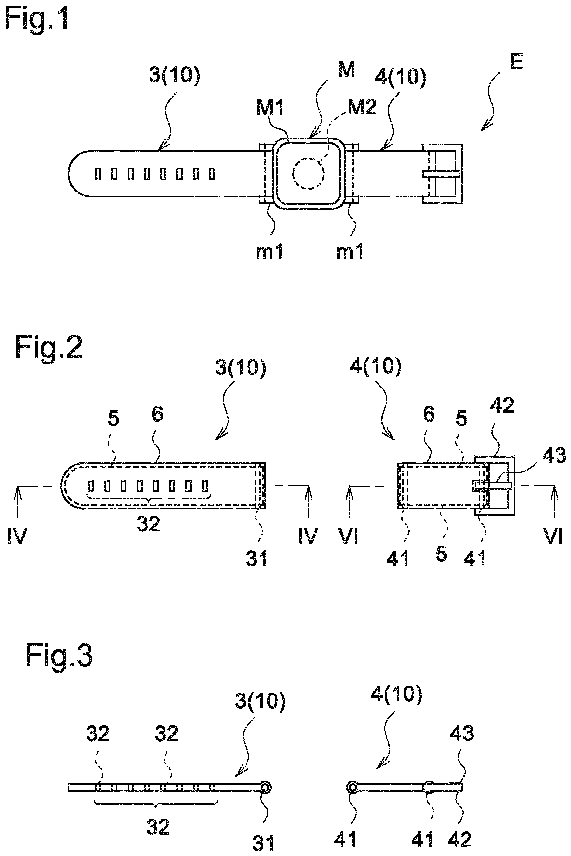

### First Embodiment [Fig. 1 to Fig. 121

A rubber band 10 according to a present embodiment will be described. Fig. 1 is a plan view of a wristwatch electronic device E including the rubber band 10. Fig. 2 is a plan view of the rubber band 10, and Fig. 3 is a front view of the rubber band 10.

The electronic device E illustrated in Fig. 1 includes an electronic device body M having a timepiece function and including a sensor that measures biological data, such a heart rate meter or pulsimeter, and the rubber band 10 that is to be held around the wrist. The electronic device body M receives, on its surface, an information display M1, which displays information such as time or biological data. The electronic device body M receives, on its back surface, a sensor M2 that comes into contact with the body to measure biological data. The sensor unit M2 is a biological sensor that radiates light through a transparent window to measure biological data. The window of the sensor M2 is disposed to face the back surface of a housing of the electronic device body M, and is in direct contact with the body.

When the electronic device body M is removed from the electronic device E, as illustrated in Figs. 2 and 3, the rubber band 10 includes two components of band bodies 3 and 4, that is, a point-side band body 3 serving as "a first band body" and a buckle-side band body 4 serving as "a second band body". Hereinbelow, the band bodies 3 and 4 refer to either one or both of the point-side band body 3 and the buckle-side band body 4. The point-side band body 3 includes a spring-bar receiving hole 31, which is an end piece through which a spring bar (not illustrated) extends to be attached to a lug m1 of the electronic device body M, and a large number of band length adjustment holes 32 into which a buckle tongue 43, described below, is fitted. The buckle-side band body 4 includes spring-bar receiving holes 41, similarly to the point-side band body 3, and the buckle tongue 43, serving as a "lock portion", which is locked on a buckle 42 and any of the band length adjustment holes 32.

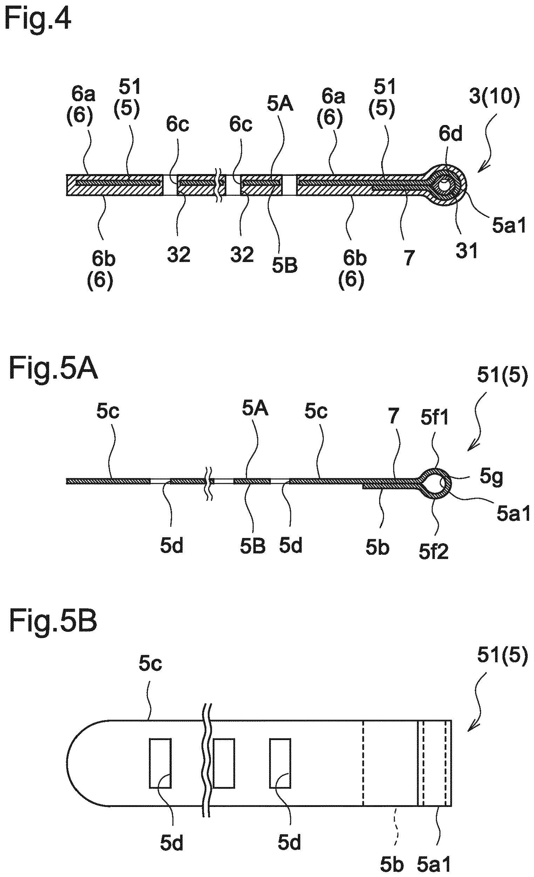

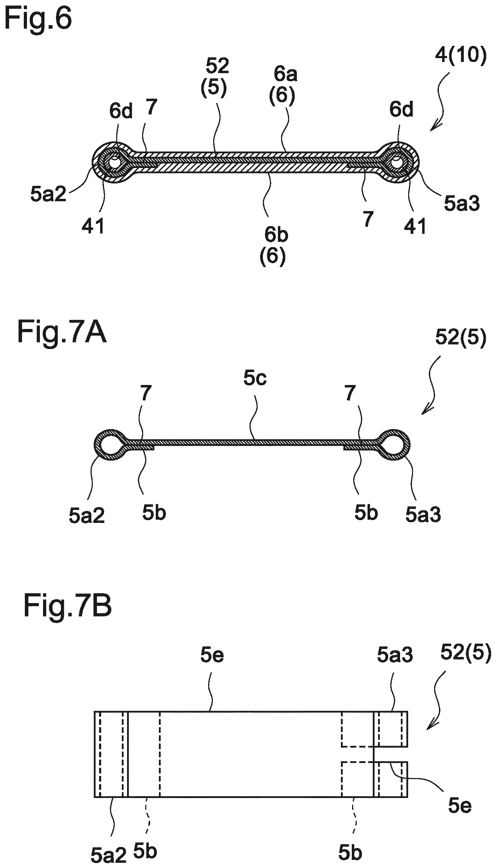

Fig. 4 is a cross-sectional view of the point-side band body 3, and Fig. 6 is a cross-sectional view of the buckle-side band body 4. As illustrated in these drawings, each of the band bodies 3 and 4 includes inside a resin portion 5 formed from a resin plate at substantially the center portion. The resin portion 5 is covered with an outer sheath 6, made of a rubber-like elastic material. The outer sheath 6 can be distinguished into an inner surface portion 6b, serving as "a second outer sheath" that comes into contact with the human body such as the wrist with the resin portion 5 interposed therebetween, and an outer surface portion 6a, serving as "a first outer sheath" that is opposite to the inner surface portion 6b. The outer surface portion 6a serving as "the first outer sheath" is formed on a surface 5A of the resin portion 5, serving as "the first surface" of the resin portion 5 (resin plate). The inner surface portion 6b serving as "the second outer sheath" is formed on a back surface 5B of the resin portion 5, serving as "the second surface" of the resin portion 5 (resin plate).

Fig. 5 illustrates a resin portion 51 inside the point-side band body 3. Of the resin portions 5, the resin portion 5 in the point-side band body 3 is referred to as the resin portion 51, and the resin portion 5 in the buckle-side band body 4, as will be described later, is referred to as a resin portion 52. The resin portion 51 inside the point-side band body 3 extends to near the outer periphery of the point-side band body 3. At the spring-bar receiving hole 31, a first cylinder 5a1 is formed by winding one end portion of the resin plate to serve as "a cylinder". An extra length portion 5b extending from the first cylinder 5a1 is layered on a flat-plate-shaped body 5c of the resin plate continuous with the first cylinder 5a. The portion where the extra length portion 5b and the body 5c are layered is preferably forms an adhesion portion 7 where the extra length portion 5b and the body 5c are fixed by, for example, thermocompression bonding, ultrasonic welding, bonding, or adhesion such as using a double-sided tape. Providing the adhesion portion 7 fixes the shape of the first cylinder 5a1, and prevents a rubber-like elastic material from intruding into the space where the body 5c and the extra length portion 5b are layered during manufacture. On the other hand, the resin portion 5 also has resin holes 5d, serving as "multiple holes", whose number corresponds to the number of the band length adjustment holes 32, at portions corresponding to the band length adjustment holes 32. The hole diameter of the resin holes 5d is larger than the hole diameter of the length adjustment holes 32.

The outer sheath 6 of the point-side band body 3 covers the entirety of the resin portion 51 inside to prevent the resin portion 51 from being exposed from the outer surface of the rubber band 10. At the spring-bar receiving hole 31, the outer sheath 6 of the point-side band body 3 covers the outer periphery of the first cylinder 5a1, while, as illustrated in Fig. 4, forming an inner-cylinder cover 6d, which covers the inner periphery of the first cylinder 5a1. On the other hand, the peripheries of the resin holes 5d corresponding to the band length adjustment holes 32 are also covered with inner-band-length-adjustment-hole covers 6c, which are part of the outer sheath 6 made of a rubber-like elastic material to hide the side surfaces of the resin holes 5d without being exposed. When the outer surface portion 6a and the inner surface portion 6b are compared in terms of thickness of the rubber-like elastic material, the inner surface portion 6b is thicker than the outer surface portion 6a in a non-layered portion where the extra length portion 5b and the body 5c are not layered since the extra length portion 5b is shorter than the body 5c. Since the rubber-like elastic material of the inner surface portion 6b that comes into contact with the body has a larger thickness, a wearer can wear the rubber band 10 while feeling flexible fitting without feeling the resin portion 51 as a hard core. Specifically, the non-layered portion is relatively soft and prevented from excessively tightly fastening the skin. The non-layered portion of the resin portion 51 is thinner than the layered portion, and thus more easily bendable, and can improve well-fitted feeling along the body (wrist).

Fig. 7 illustrates the resin portion 52 inside the buckle-side band body 4. The resin portion 52 inside the buckle-side band body 4 extends to near the outer periphery of the buckle-side band body 4. At each of the spring-bar receiving holes 41 at an end piece, a second cylinder 5a2 and a third cylinder 5a3 are formed in the buckle-side band body 4, as in the case of the spring-bar receiving hole 31 formed in the point-side band body 3. The second cylinder 5a2 serves as "a cylinder", formed by winding an end portion of the resin plate. The second cylinder 5a2 has a length extending throughout in the width direction of the resin plate. The third cylinder 5a3 has a cutout 5e, into which the buckle tongue 43 is inserted, at the center of the third cylinder 5a3 by winding a pair of pieces at the end portion of the resin plate. The third cylinder 5a3 is different from the second cylinder 5a2 and the first cylinder 5a1 of the point-side band body 3 in that it has the cutout 5e. The buckle-side band body 4 has no band adjustment holes 32, and the resin portion 52 thus has no resin holes 5d.

The outer sheath 6 of the buckle-side band body 4 also covers the entirety of the resin portion 52 inside, not to expose the resin portion 52. Also at the spring-bar receiving holes 41, the inner-cylinder covers 6d are formed to cover the inner peripheral surfaces of the second cylinder 5a2 and the third cylinder 5a3, not only the outer peripheral surfaces of the second cylinder 5a2 and the third cylinder 5a3. When the outer surface portion 6a and the inner surface portion 6b are compared in terms of thickness of the rubber-like elastic material, the inner surface portion 6b is thicker than the outer surface portion 6a at a non-layered portion where the extra length portions 5b and the body 5c are not layered, since the extra length portions 5b are shorter than the body 5c. Since the rubber-like elastic material of the inner surface portion 6b that comes into contact with the body has a larger thickness, a wearer can wear the rubber band 10 while feeling flexible fitting without feeling the resin portion 52 as a hard core. Specifically, the non-layered portion is relatively soft and is prevented from excessively tightly fastening the skin. The non-layered portion of the resin portion 52 is thinner than the layered portion, and thus more easily bendable, and can improve well-fitted feeling along the body (wrist).

The resin plate forming the resin portion 5 may be formed from, for example, polyester (polyethylene terephthalate (PET) or polyethylene naphthalate (PEN)), polyethylene, polypropylene, polycarbonate, polyamide, polyimide, ethylene-vinyl acetate copolymer, polyether sulfone, polyvinyl chloride, polyarylate, polymethyl methacrylate, polyacetal, polyurethane, polyurea, polystyrene, acrylonitrile-butadienestyrene (ABS) resin, epoxy resin, or phenol resin. These resins are thermally resistant, less stretchable, and highly strong, and thus are suitable as the material of the resin portion 5. From these viewpoints, the resin plate is preferably a biaxial stretching film.

The resin plate forming the resin portion 5 may contain a reinforcing material. Examples of the reinforcing material include glass fiber, carbon fiber, cloth, resin mesh, or wire mesh. Instead, a sheet formed from glass fiber, carbon fiber, cloth, resin mesh, or wire mesh layered on and fixed to the surface of the resin plate may be used. The reinforcing material may be used after undergoing surface treatment with, for example, a coupler or a primer. A fibrous material forming the reinforcing material is installed inside the resin plate or fixed to the surface of the resin plate, and thus hardly ever loosened or disentangled like a simple cloth. Alternatively, a material formed by fixing (for example, welding or bonding) fiber threads forming the reinforcing material may be used.

The surface of the resin plate can be treated with various types of surface treatment. Undergoing surface treatment enhances adhesion properties of the resin plate with the outer sheath 6. Examples of surface treatment include corona treatment, plasma treatment, ultraviolet irradiation treatment, excimer-laser irradiation treatment, primer application, sandblasting, and embossing roller processing.

The resin plate forming the resin portion 5 preferably has a thickness of 50 µm to 500 µm, or more preferably, 70 µm to 300 µm. The resin plate thinner than 50 µm has a small reinforcing effect on the outer sheath 6, and may make the outer sheath 6 easily cuttable. The resin plate thicker than 500 µm has flexibility insufficient to serve as the rubber band 10, and may make the rubber band 10 less easily bendable. Specifically, the resin plate having a thickness of within the range of 50 µm to 500 µm is easily bendable without impairing the bendability of the rubber band 10, and makes the rubber band 10 easily bendable. The resin plate having a thickness of within the range of 70 µm to 300 µm is easily bendable while being less cuttable.

The resin portion 5 also needs to have holes at portions corresponding to the point-side band body 3 of the band length adjustment holes 32. The resin holes 5d preferably have a hole diameter of 1.1 mm to 6 mm, and adjacent resin holes 5d are preferably spaced apart from each other by 0.2 mm to 4.8 mm. By forming the resin holes 5d of the resin portion 5 having a larger hole diameter than the hole diameter of the band length adjustment holes 32 of the rubber band 10, the outer sheath 6 can be also disposed on the inner side of the resin holes 5d to form the inner-band-length-adjustment-hole cover 6c, so that the inner peripheral surfaces of the resin holes 5d are covered.

Examples of the rubber-like elastic material forming the outer sheath 6 include synthetic rubber such as silicone rubber, polyurethane rubber, styrene-butadiene rubber, butadiene rubber, ethylene-propylene rubber, acrylic rubber, or fluorocarbon rubber, and thermo-plastic elastomer such as styrene thermo-plastic elastomer, olefin thermo-plastic elastomer, or polyurethane thermo-plastic elastomer. Among these, silicone rubber is preferable as having thermal resistance, weather resistance, and chemical resistance, and having low compression permanent strain. The hardness of such a rubber-like elastic material is preferably within the range of 30 to 80, or more preferably 40 to 60, when measured by a type A durometer specified in JIS K6253. The material having a hardness of smaller than 30 when measured by a type A durometer is too soft, and may be cut at a periphery or end portion that is not reinforced with the resin portion 5. On the other hand, the material having a hardness larger than 80 when measured by a type A durometer is too hard to provide a wearer with ill-fitting feeling.

By thus covering with the outer sheath 6 both surfaces of the first cylinder 5a1, the second cylinder 5a2, and the third cylinder 5a3 formed from resin, at the portions corresponding to the spring-bar receiving holes 31 and 41, the spring-bar receiving holes 31 and 41 become less cuttable, and the friction resistance inside and outside the spring-bar receiving holes 31 and 41 is enhanced, and the outer appearance is improved. The first cylinder 5a1, the second cylinder 5a2, and the third cylinder 5a3 are fixed by winding the resin plate into a roll shape. Thus, when integrally molded with the outer sheath 6 during manufacture, the resin plate can be easily held in a mold (easily positioned), so that molding errors such as the resin plate partially protruding from the outer sheath 6 to be exposed to the outside are less likely to occur.

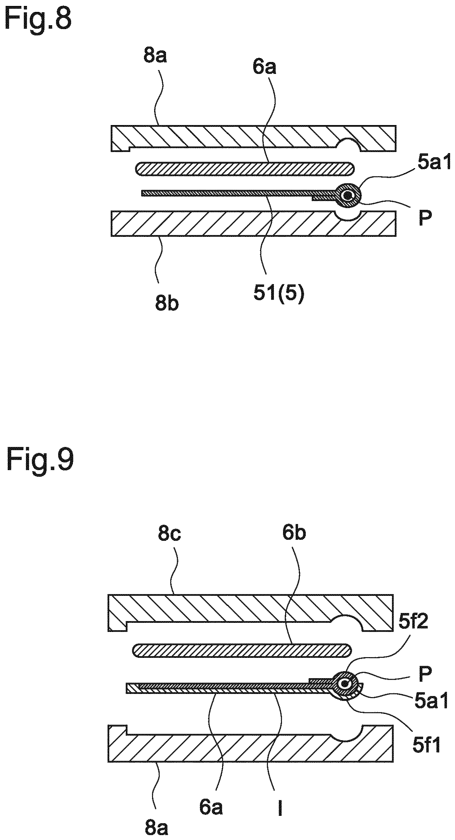

A method for manufacturing the rubber band 10 will be described. The rubber band 10 has the resin portion 5 located inside the outer sheath 6. Positioning the resin portion 5 at the center of the rubber band 10 with a single step is difficult. Thus, the rubber band 10 is preferably manufactured with two molding steps. To integrally mold the resin portion 5 and the outer sheath 6, the resin portion 5 and the outer sheath 6 are molded with different molds (first mold and second mold) for the point-side band body 3 and the buckle-side band body 4. Although the molds have different shapes, the manufacturing steps are the same. Thus, the point-side band body 3 will be described below by way of example with reference to Fig. 8 to Fig. 12 without describing the buckle-side band body 4.

First, the resin portion 5 is molded in a predetermined shape. Specifically, the resin plate forming the resin portion 51 of the point-side band body 3 is cut into the shape and size as illustrated in Fig. 5, the resin holes 5d are formed at portions corresponding to the band length adjustment holes 32, and an end portion forming an end piece is wound to form the first cylinder 5a1. The adhesion portion 7 is formed as needed at the portion where the extra length portion 5b and the body 5c are layered. When the resin portion 52 is formed on the buckle-side band body 4, the resin portion 52 forms the second cylinder 5a2 and the third cylinder 5a3. As illustrated in Fig. 7B, the third cylinder 5a3 has the cutout 5e at a portion corresponding to the buckle tongue 43. Thus, the end portion of the resin plate cut to have a recess or a rectangular or ellipse opening at the middle in advance needs to be wound to form the third cylinder 5a3.

Subsequently, the resin portion 5 and the outer sheath 6 are integrally molded. To this end, firstly, one surface of the resin portion 5, that is, either one of the outer surface portion 6a and the inner surface portion 6b is integrally molded with the resin portion 5. Although the inner surface portion 6b may be molded first, here, the example where the outer surface portion 6a is molded first will be described. In this example, the outer surface portion 6a serves as a first outer sheath, and the inner surface portion 6b serves as a second outer sheath. In the case where the inner surface portion 6b is formed first, the inner surface portion 6b serves as a first outer sheath, and the outer surface portion 6a serves as a second outer sheath.

As illustrated in Fig. 8, an uncured rubber-like elastic material that forms the outer surface portion 6a when cured, and the resin plate 51 are inserted into an outer-surface mold 8a, which molds the outer surface portion 6a of the rubber band 10. The rubber-like elastic material is disposed on the surface 5A of the resin plate 51. A back mold 8b, which supports an outer-surface mold 8 and a back surface 5B of the resin portion 51 is clamped to mold the outer surface portion 6a with the rubber-like elastic material. Here, a metal shaft P having a smaller diameter than the hole diameter of the first cylinder 5a1 is inserted into the first cylinder 5a1, and placed in the molds 8a and 8b. The metal shaft P also helps positioning of the resin portion 51 in the back mold 8b. In this first step, the outer surface portion 6a formed from the rubber-like elastic material and the surface 5A of the resin portion 5 are layered to be integrated, and the outer surface portion 6a is formed to cover an outer peripheral surface 5f1 of the first cylinder 5a1. Thus, an intermediate product I obtained by forming the outer surface portion 6a formed from the rubber-like elastic material on the surface 5A of the resin portion 51 is obtained (Fig. 9). In this example, the outer-surface mold 8a and the back mold 8b correspond to "a first mold".

The second step is a step of forming the inner surface portion 6b on the intermediate product I. In place of the back mold 8b described above, an inner-surface mold 8c for forming the inner surface portion 6b is prepared. As illustrated in Fig. 9, an uncured rubber-like elastic material serving as the material of the inner surface portion 6b and the intermediate product I are inserted into the inner-surface mold 8c, and they are molded between the outer-surface mold 8a and the inner-surface mold 8c. As in the case of the first step, also in this second step, an uncured rubber-like elastic material is clamped to be molded. Thus, the rubber-like elastic material is prevented from excessively flowing to the outer surface portion 6a. In this second step, the inner surface portion 6b is formed on the back surface 5B of the resin portion 51, which is not formed in the first step. Thus, the inner surface portion 6b is formed to cover an outer peripheral surface 5f2 of the first cylinder 5a1. Thus, the point-side band body 3 in which the resin portion 51 is integrated with the outer surface portion 6a while being held between the outer surface portion 6a and the inner surface portion 6b is obtained. The buckle-side band body 4 is similarly manufactured, so that the rubber band 10 including the point-side band body 3 and the buckle-side band body 4 is obtained. In this example, the outer-surface mold 8a and the inner-surface mold 8c correspond to "a second mold".

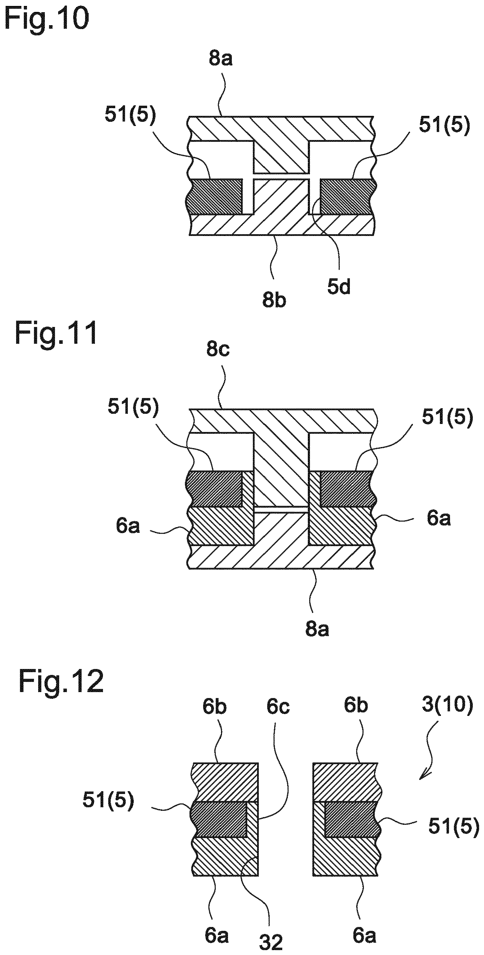

In the manufacturing method for integrally molding the resin portion 51 and the outer sheath 6, molding the band length adjustment holes 32 will be additionally described. Fig. 10 is an enlarged schematic diagram around one of the resin holes (or simply "hole") 5d when the resin portion 51 is placed in the molds 8a and 8b for being molded. Firstly, the first step is performed, where the resin portion 51 is inserted between the back mold 8b and the outer-surface mold 8a to integrally mold the resin portion 51 and the outer surface portion 6a. When the first cylinder 5a1 of the resin portion 51 is positioned with respect to the back mold 8b, a gap is left between the back mold 8b and the resin holes 51, as illustrated in Fig. 10. Thus, in the first step, the inner-band-length-adjustment-hole cover 6c, which is the rubber-like elastic material covering the side surfaces of the resin holes 5d, can be formed.

In the second step where the intermediate product I obtained in the first step and the inner surface portion 6b are integrally formed, the intermediate product I can be fitted into the outer-surface mold 8a, and thus can be positioned with the fitting, as illustrated in Fig. 11. The intermediate product I is molded with the inner-surface mold 8c in place of the back mold 8a, between the inner-surface mold 8c and the outer-surface mold 8a, so that the point-side band body 3 illustrated in Fig. 12, in which the peripheries of the resin holes 5d are covered with the rubber-like elastic material, is formed.

In the above manufacturing method, the uncured rubber-like elastic material is inserted into the mold 8 (8a, 8b, 8c) instead of undergoing injection molding. Thus, placing the uncured rubber-like elastic material on top of the resin plate does not cause the uncured rubber-like elastic material to excessively flow in the mold 8. The uncured rubber-like elastic material that is to enter the inner portions defined by the inner peripheral surfaces 5g of the first cylinder 5a1, the second cylinder 5a2, and the third cylinder 5a3 fills only the upper half of the resin plate. Thus, when the inner surface portion 6b is to be molded in the second step, the uncured rubber-like elastic material enters the upper side of the inner portions defined by the inner peripheral surfaces 5g of the first cylinder 5a1, the second cylinder 5a2, and the third cylinder 5a3, so that the inner-cylinder covers 6d can be formed in the inner portions defined by the first cylinder 5a1, the second cylinder 5a2, and the third cylinder 5a3 with these two steps.

The above manufacturing method requires two types of molds, that is, the back mold 8b and the inner-surface mold 8c corresponding to the inner surface portion 6b of the rubber band 10, whereas requiring a single type of the outer mold 8a. Thus, the above manufacturing method reduces the costs for the molds even requiring two steps.

The rubber band 10 thus manufactured has the band bodies 3 and 4 having a thickness of 2 mm to 5 mm. The band length adjustment holes 32 of the point-side band body 3 have a hole diameter of 1 mm to 5 mm, and the distance between the band length adjustment holes 32 can be determined as 0.5 mm to 5 mm. Although the distance between the band length adjustment holes 32 is reduced, the resin portion 5 formed from the resin plate and disposed between the adjacent band length adjustment holes 32 reinforces the portions between the adjacent band length adjustment holes 32 to prevent the portions from being elongated or cut.

The rubber band 10 has the side surface of the resin plate and the inner surfaces of the band length adjustment holes 32 covered with the rubber-like elastic material. Thus, the rubber band 10 can have fine appearance and flexibility on both outer and back surfaces. The rubber band 10 allows the electronic device body M to come into contact with the human body, and is less easily elongated or cut. Thus, a wearer can continuously wear the electronic device body M while being in desirable contact with his/her body. The rubber band 10 has the resin portion 5 disposed also at portions between the band length adjustment holes 32. Thus, the portions between the band length adjustment holes 32 are less likely to be cut or elongated.

In the manufacturing method according to the present embodiment, the outer surface portion 6a is integrated with the resin portion 5 earlier than the inner surface portion 6b. Thus, the inner surface portion 6b and the outer surface portion 6a may be formed from different materials having different physical properties. For example, the inner surface portion 6b is a portion that comes into contact with the skin, and is thus preferably formed from a soft and elastic material. The outer surface portion 6a is away from the skin, and needs to be scratchproof or solid in case of being bumped or fallen. The material of the outer surface portion 6a is thus preferably harder and stronger than the material of the inner surface portion 6b. By molding the hard and strong material first and then molding the soft material, the inner surface portion 6b formed from the soft rubber-like elastic material can be manufactured while retaining yield without causing a mold shift.

In the above manufacturing method, as to molding of the portions corresponding to the band length adjustment holes 32, in the first step where the resin portion 51 and the outer surface portion 6a are integrally formed, the inner-band-length-adjustment-hole cover 6c is formed by covering the side surfaces of the resin holes 5d with the rubber-like elastic material covers. Instead, in the second step where the resin portion 51 and the inner surface portion 6b are integrally formed, the inner-band-length-adjustment-hole cover 6c may be formed by covering the side surfaces of the resin holes 5d with the rubber-like elastic material. Alternatively, in each of integral molding of the first step and the second step, the inner-band-length-adjustment-hole cover 6c covering half the resin portion 51 in depth may be formed.

According to the above rubber band 10 and the method for manufacturing the same, the rubber band 10 allows the electronic device body M to come into contact with the body as appropriate. In addition, the rubber band 10 can be made to have a fine texture, be less cuttable, and have a fine external design.

### Second Embodiment [Fig. 131

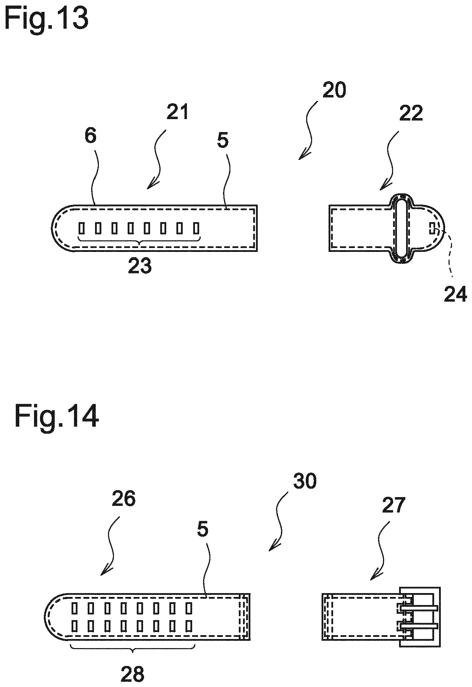

Fig. 13 is a plan view of a rubber band 20 according to the present embodiment. The rubber band 20 has no buckle, and connects a second band body 22 (second band body) to a first band body 21 (first band body) with a button 24. The first band body 21 corresponding to the point-side band body 3 has multiple button receiving grooves 23, serving as "band length adjustment holes" instead of the band length adjustment holes 32. The second band body 22 corresponding to the buckle-side band body 4 has the button 24, serving as "a lock portion", instead of the buckle 42 and the buckle tongue 43. No spring-bar receiving hole is disposed on the side connected to the electronic device body M.

The rubber band 20 also has a resin portion 5 formed from a resin plate at substantially the center of the inside. The rubber band 20 has the outer side covered with the outer sheath 6 formed from the rubber-like elastic material. As in the case of the rubber band 10, the outer sheath 6 includes an inner surface portion 6b, which comes into tight contact with the wrist or other parts with the resin portion 5 interposed between, and the outer surface portion 6a, which is the surface opposite to the inner surface portion 6b. The outer sheath 6 and the resin portion 5 can be integrally molded in the same manner as in the case of the rubber band 10.

The button receiving grooves 23 do not extend through the rubber band 20. The button receiving grooves 23 are formed in the inner surface portion 6b, without being exposed from the outer surface portion 6a. The button receiving grooves 23 have a depth that is half the depth of the first band body 21 or larger. As in the case of the rubber band 10, the resin portion 5 disposed at substantially the center is disposed around the button receiving grooves 23. Portions of the first band body 21 and the second band body 22 that are connected to the electronic device body M may have an attachment structure compatible with an attachment structure of the rubber band 20 disposed on the electronic device body M to which the rubber band 20 is connected. As in the case of the button 24 formed from metal or resin, such an attachment structure can be attached to the first band body 21 or the second band body 22 after the resin portion 5 and the outer sheath 6 are integrally molded.

### Third Embodiment [Fig. 141

Fig. 14 is a plan view of a rubber band 30 according to the present embodiment. A first band body 26 corresponding to the point-side band body 3 includes two rows of band length adjustment holes 28. The resin portion 5 preferably has two rows of resin holes 5d along the band length adjustment holes 28.

The above embodiments are examples of the present invention. Within the scope not departing from the gist of the present invention, the embodiments may be changed, may have known techniques added, or may be combined one with another, and such technologies are also included in the scope of the present invention.

### Reference Signs List

- 10: rubber band (first embodiment)
- 3: point-side band body (first band body)
- 31: spring-bar receiving hole
- 32: band length adjustment hole
- 4: buckle-side band body (second band body)
- 41: spring-bar receiving hole
- 42: buckle
- 43: buckle tongue (lock portion)
- 5: resin portion (resin plate)
- 5A: surface (first surface)
- 5B: back surface (second surface)
- 51: resin portion of point-side band body
- 52: resin portion of buckle-side band body
- 5a1: first cylinder (cylinder)
- 5a2: second cylinder (cylinder)
- 5a3: third cylinder (cylinder)
- 5b: extra length portion
- 5c: body
- 5d: resin hole (hole)
- 5e: cutout
- 5f1, 5f2: outer peripheral surface of cylinder
- 5g: inner peripheral surface of cylinder
- 6: outer sheath
- 6a: outer surface portion
- 6b: inner surface portion
- 6c: inner-band-length-adjustment-hole cover
- 6d: inner-cylinder cover
- 7: adhesion portion
- 8: mold
- 8a: outer-surface mold
- 8b: back mold
- 8c: inner-surface mold
- E: electronic device (wearable electronic device)
- M: electronic device body
- m1: lug
- I: intermediate product
- P: metal shaft
- 20: rubber band (second embodiment)
- 21: first band body (first band body)
- 22: second band body (second band body)
- 23: button receiving groove
- 24: button (lock portion)
- 25: lug mount
- 30: rubber band (third embodiment)
- 26: first band body (first band body)
- 27: second band body (second band body)
- 28: band length adjustment hole

## Claims

1. A method for manufacturing a rubber band including a band body attached to an electronic device body and worn by a human body, the band body including
a resin portion formed from a resin plate having a plurality of holes,
an outer sheath made of a rubber-like elastic material that covers the resin portion, and
a plurality of band length adjustment holes that extend through the plurality of holes of the resin portion and the outer sheath, the method comprising:
forming a first outer sheath covering a first surface of the resin plate by inserting the resin plate into a first mold, and
subsequently forming a second outer sheath covering a second surface of the resin plate by inserting the resin plate on which the first outer sheath is disposed into a second mold.

2. The method for manufacturing a rubber band according to Claim 1,
wherein when forming the first outer sheath or the second outer sheath, the band length adjustment holes are formed by covering an inner peripheral surface of each of the plurality of holes with the rubber-like elastic material.

3. The method for manufacturing a rubber band according to Claim 1 or 2,
wherein the resin plate includes
a flat plate-shaped body,
a cylinder formed by winding an end portion of the body, and
an extra length portion extended from the cylinder and layered on the body, and
wherein when forming each of the first outer sheath and the second outer sheath, an outer peripheral surface of the cylinder is partially covered.

4. The method for manufacturing a rubber band according to Claim 3,
wherein when forming the first outer sheath or the second outer sheath, an inner peripheral surface of the cylinder is covered with the rubber-like elastic material.

5. The method for manufacturing a rubber band according to any one of Claims 1 to 4,
wherein the first mold and the second mold form an uncured rubber-like elastic material and the resin portion.

6. A rubber band comprising a first band body attached to an electronic device body and worn by a human body,
wherein the first band body includes
a plate-shaped resin portion having a plurality of holes, and
an outer sheath made of a rubber-like elastic material that covers the resin portion,
wherein the outer sheath includes
an outer surface portion that covers a first surface of the resin portion, and
an inner surface portion that covers a second surface of the resin portion and comes into contact with a human body,
wherein the outer surface portion and the inner surface portion are formed as separate bodies,
wherein the first band body further includes a plurality of band length adjustment holes into which the lock portion is inserted, and
wherein each of the band length adjustment holes has an inner-band-length-adjustment-hole cover formed by covering an inner peripheral surface of each band length adjustment hole with the outer sheath.

7. The rubber band according to Claim 6, further comprising:
a second band body different from the first band body,
wherein the second band body includes a lock portion that is locked with the first band body.

8. The rubber band according to Claim 6 or 7,
wherein the resin portion includes a flat plate-shaped body, a cylinder formed by winding an end portion of the body, and an extra length portion extended from the cylinder and layered on the body.

9. The rubber band according to Claim 8,
wherein the outer sheath includes an inner-cylinder cover that covers an inner peripheral surface of the cylinder.

10. A wearable electronic device, comprising:
an electronic device body; and
the rubber band according to any one of Claims 5 to 9.
